# EUROPÄISCHE PATENTSCHRIFT

(11) **EP 2 827 753 B1**
(45) Veröffentlichungstag und Bekanntmachung des Hinweises auf die Patenterteilung: **27.12.2017**
(21) Anmeldenummer: 12718057.8
(22) Anmeldetag: 21.03.2012
(51) Int. Cl.: A47J 37/12

(54) **FRITTIEREINRICHTUNG UND VERFAHREN ZUR KONTROLLE EINER FRITTIEREINRICHTUNG**
DEEP-FRYING DEVICE AND METHOD FOR MONITORING A DEEP-FRYING DEVICE
DISPOSITIF DE FRITURE ET PROCÉDÉ DE COMMANDE D'UN DISPOSITIF DE FRITURE

(43) Veröffentlichungstag der Anmeldung: 28.01.2015
(73) Patentinhaber: Testo SE & Co. KGaA, 79853 Lenzkirch (DE)
(72) Erfinder: BRUGGER, Simon, 79853 Lenzkirch (DE)
(74) Vertreter: Börjes-Pestalozza, Henrich
(86) Internationale Anmeldenummer: PCT/EP2012/001303
(87) Internationale Veröffentlichungsnummer: WO 2013/139354

(56) Entgegenhaltungen:
- EP-A1- 1 439 388
- EP-A1- 2 221 611
- US-A- 5 818 731

## Beschreibung

Die Erfindung betrifft eine Frittiereinrichtung mit einem Frittierölbecken, das zur Aufnahme von Frittieröl eingerichtet ist, einem stationären Frittierölsensor, welcher zur Messung einer elektrischen Eigenschaft des in dem Frittierölbecken aufgenommenen Frittieröls eingerichtet ist, und einer an den stationären Frittierölsensor angeschlossenen stationären Auswerteeinheit, mit welcher ein Messsignal des stationären Frittierölsensors automatisch mittels wenigstens einer in der stationären Auswerteeinheit hinterlegten Kennlinie in eine Bewertungsgröße zur Charakterisierung des Frittieröls umwandelbar ist.

Die Erfindung betrifft schließlich ein Verfahren zur Kontrolle einer Frittiereinrichtung der eingangs beschriebenen Art, wobei die Frittiereinrichtung einen stationären Frittierölsensor, welcher zur Messung einer elektrischen Eigenschaft eines in einem Frittierölbecken der Frittiereinrichtung aufgenommenen Frittieröls eingerichtet ist, aufweist und wobei in einer an den stationären Frittierölsensor angeschlossenen stationären Auswerteeinheit ein Messsignal des stationären Frittierölsensors automatisch mittels wenigstens einer in der stationären Auswerteeinheit hinterlegten Kennlinie in eine Bewertungsgröße zur Charakterisierung des Frittieröls umwandelbar ist.

Frittiereinrichtungen der beschriebenen Art sind bekannt, siehe z.B. US5818731A, und werden als sogenannte Fritteusen zum Frittieren von Speisen eingesetzt. Der stationäre Frittierölsensor dient dazu, den Verbrauchtheitsgrad des eingesetzten Frittieröls zu ermitteln. Dieser Verbrauchtheitsgrad kann beispielsweise durch die Bewertungsgröße %TPM (Total Polar Materials) oder %FFA (free fatty acids, freie Fettsäuren)gegeben sein.

Jeder stationäre Frittierölsensor muss nach dem Herstellungsprozess individuell abgeglichen werden. Dies erfolgt idealerweise im Medium Öl.

Zusätzlich muss ein stationärer Frittierölsensor aufgrund von Alterung, Verunreinigung, geometrischer Veränderung oder Beschädigung in regelmäßigen Abständen kalibriert oder justiert werden, um im Einsatz zuverlässige Messwerte zu liefern. Werden diese Kalibrierungen oder Justagen nicht durchgeführt, so ergeben sich bei der Messung mit dem Frittierölsensor Messfehler, die die ordnungsgemäße Funktion der Frittiereinrichtung beeinträchtigen.

Der Erfindung liegt die Aufgabe zugrunde, die Kontrolle der ordnungsgemäßen Funktion einer Frittiereinrichtung zu vereinfachen.

Zur Lösung dieser Aufgabe ist bei einer Frittiereinrichtung der eingangs beschriebenen Art erfindungsgemäß vorgesehen, dass in der wenigsten einen Kennlinie wenigstens ein Referenzpunkt definiert oder definierbar ist und dass eine Empfangseinheit ausgebildet ist, welche eine Empfangs-Datenschnittstelle zum Empfang eines Referenzwertes für den Referenzpunkt bereitstellt. Von Vorteil ist dabei, dass auf diese Weise automatisiert ein Abgleich der wenigstens einen hinterlegten Kennlinie und somit eine Kontrolle der ordnungsgemäßen Funktion des stationären Frittierölsensors durchführbar ist. Insbesondere ist so eine Kontrolle einer großen Anzahl von Frittiereinrichtungen in verhältnismäßig kurzer Zeit durchführbar. Wegen des geringen Zeitaufwands, den die Übermittlung eines Referenzwertes an die Frittiereinrichtung erfordert, kann die Kontrolle auch in deutlich verkürzten Zeitabständen erfolgen. Dies vereinfacht die Kontrolle der ordnungsgemäßen Funktion der Frittiereinrichtung.

Bei einer Ausgestaltung der Erfindung kann vorgesehen sein, dass der stationäre Frittierölsensor in einem Aufnahmeraum des Frittierölbeckens für das Frittieröl angeordnet ist. Von Vorteil ist dabei, dass mit dem stationären Frittierölsensor der Verbrauchtheitsgrad und/oder die Qualität des eingesetzten Frittieröls im Betrieb der Frittiereinrichtung bestimmbar ist.

Es kann vorgesehen sein, dass der stationäre Frittierölsensor an dem Frittierölbecken befestigt ist. Dies verbessert die Gebrauchseigenschaften der Frittiereinrichtung, da der stationäre Frittierölsensor nicht verloren gehen kann.

Die Datenschnittstelle kann zur drahtgebundenen Datenübertragung des Referenzwertes ausgebildet sein. Besonders günstig ist es jedoch, wenn die Empfangs-Datenschnittstelle zur drahtlosen Datenübertragung eingerichtet ist. Beispielsweise kann die Datenübertragung per Funk, per Infrarot-Strahlung, induktiv, kapazitiv oder auf andere Weise eingerichtet sein. Von Vorteil ist dabei, dass die Frittiereinrichtung besser reinigbar ist.

Bei einer Ausgestaltung der Erfindung kann vorgesehen sein, dass der Referenzwert während eines Messbetriebs der stationären Auswerteeinheit empfangbar ist. Von Vorteil ist dabei, dass eine Kontrolle der ordnungsgemäßen Funktion der Frittiereinrichtung während des normalen Betriebs durchführbar ist.

Bei einer Ausgestaltung der Erfindung kann vorgesehen sein, dass jeder Punkt der wenigstens einen Kennlinie als Referenzpunkt verwendbar oder definierbar ist. Von Vorteil ist dabei, dass die Kontrolle der ordnungsgemäßen Funktion unabhängig von den Eigenschaften des eingefüllten Frittieröls und von sonstigen Betriebsbedingungen durchführbar ist. Die Verwendung eines standardisierten Referenz-Frittieröls bei einer vorgegebenen Temperatur ist somit verzichtbar.

Es kann vorgesehen sein, dass der Referenzpunkt durch das aktuelle Messsignal des stationären Frittieröls definiert ist. Dies ermöglicht eine besonders einfache Kontrolle der ordnungsgemäßen Funktion der Frittiereinrichtung und insbesondere des stationären Frittierölsensors im Betrieb.

Bei einer Ausgestaltung der Erfindung kann vorgesehen sein, dass eine Vergleichseinheit ausgebildet ist, die zur Generierung eines Hinweissignals, wenn der Referenzwert um wenigstens einen Toleranzwert von einem Wert der hinterlegten Kennlinie am Referenzpunkt abweicht, eingerichtet ist. Alternativ oder zusätzlich kann vorgesehen sein, dass eine Vergleichseinheit ausgebildet ist, die zur Generierung eines Hinweissignals, wenn der Referenzwert um wenigstens einen Toleranzwert von einem aus einem Messsignal des stationären Frittierölsensors gebildeten Wert der Bewertungsgröße abweicht, eingerichtet ist. Somit ist eine nicht-ordnungsgemäße Funktion des stationären Frittierölsensors einfach und direkt erkennbar und anzeigbar bzw. ausgebbar.

Bei einer Ausgestaltung der Erfindung kann vorgesehen sein, dass die wenigstens eine Kennlinie modifizierbar hinterlegt ist. Von Vorteil ist dabei, dass in der stationären Auswerteeinheit eine zeitliche Änderung der Funktionseigenschaften des stationären Frittierölsensors einfach kompensierbar sind, indem die wenigstens eine Kennlinie derart modifiziert wird, dass sich zumindest näherungsweise oder sogar exakt das ursprüngliche Messverhalten des stationären Frittierölsensors ergibt.

Bei einer Ausgestaltung der Erfindung kann vorgesehen sein, dass die stationäre Auswerteeinheit ein Mittel zur Modifizierung der wenigstens einen hinterlegten Kennlinie aufweist, mit welchem die wenigstens eine Kennlinie derart modifizierbar ist, dass ein Kennlinienwert der modifizierten wenigstens einen Kennlinie am Referenzpunkt gleich dem Referenzwert ist. Von Vorteil ist dabei, dass eine Kompensation von Veränderungen am stationären Frittierölsensor aufgrund von Alterungsprozessen oder mechanischen Veränderungen automatisiert kompensierbar ist.

Bei einer Ausgestaltung der Erfindung kann vorgesehen sein, dass der stationäre Frittierölsensor und/oder die stationäre Auswerteeinheit zum Betrieb bei unterschiedlichen Arbeitsfrequenzen des stationären Frittierölsensors (15) eingerichtet ist/sind. Von Vorteil ist dabei, dass Multifrequenzmessungen der frequenzabhängigen Permittivität durch Anfahren unterschiedlicher Arbeitsfrequenz durchführbar sind. Hierdurch ist die Genauigkeit der Charakterisierung des Frittieröls verbesserbar.

Hierbei kann vorgesehen sein, dass mehrere, unterschiedlichen Arbeitsfrequenzen des stationären Frittierölsensors zugeordnete Kennlinien zur Verwendung in der stationären Auswerteeinheit hinterlegt sind. Somit sind frequenzabhängige Auswertungen von Ausgangssignalen des stationären Frittierölsensors ausführbar.

Bei einer Ausgestaltung der Erfindung kann vorgesehen sein, dass mit dem stationären Frittierölsensor eine von der Permittivität des Frittieröls abhängige physikalische Größe messbar ist. Bevorzugt werden der Realteil und/oder der Imaginärteil und/oder der Betrag der Permittivität bei einer vorgegebenen Frequenz kapazitiv oder auf andere Weise gemessen. Somit kann auf einfache Weise der Verbrauchtheitsgrad des eingesetzten Frittieröls ermittelt werden.

Beispielsweise kann hier vorgesehen sein, dass der stationäre Frittierölsensor als kapazitiver Messsensor ausgebildet ist.

Um den Referenzwert bei Bedarf empfangen zu können, kann vorgesehen sein, dass die Frittiereinrichtung ein Mittel zum Verbindungsaufbau einer Datenverbindung über die Datenschnittstelle aufweist. Bei einem portablen Frittierölmessgeräts der eingangs genannten Art ist vorgesehen, dass eine Sendeeinheit ausgebildet ist, welche eine Sende-Datenschnittstelle zum Senden eines aus dem Messsignal des portablen Frittierölsensors als Bewertungsgröße bei der Umwandlung gebildeten Referenzwertes bereitstellt. Von Vorteil ist dabei, dass das portable Frittierölmessgerät eine unabhängige Messung bereitstellt, mit welcher die ordnungsgemäße Funktion der Frittiereinrichtung und des stationären Frittierölsensors der Frittiereinrichtung kontrollierbar ist.

Bei einer Ausgestaltung kann vorgesehen sein, dass mit dem portablen Frittierölsensor eine von der Permittivität des Frittieröls abhängige physikalische Größe messbar ist, insbesondere dass der portable Frittierölsensor als kapazitiver Sensor ausgebildet ist. Von Vorteil ist dabei, dass mit dem portablen Frittierölsensor eine Aussage über den Verbrauchtheitsgrad des eingesetzten Frittieröls gewinnbar ist, welcher als Referenzwert zur Kontrolle der ordnungsgemäßen Funktion des Frittiergeräts verwendbar ist.

Bei einer Ausgestaltung kann vorgesehen sein, dass der portable Frittierölsensor über ein stabförmiges Verbindungselement an der portablen Auswerteeinheit befestigt ist. Von Vorteil ist dabei, dass der portable Frittierölsensor einfach in heißes Frittieröl eintauchbar ist, ohne dass die portable Auswerteeinheit Schaden nimmt.
Um einen Kontrollvorgang in einer Frittiereinrichtung auslösen zu können, kann vorgesehen sein, dass das portable Frittierölmessgerät ein Mittel zum Verbindungsaufbau einer Datenverbindung über die Sende-Datenschnittstelle aufweist.

Bei einer Ausgestaltung kann vorgesehen sein, dass der portable Frittierölsensor und/oder die portable Auswerteeinheit zum Betrieb bei unterschiedlichen Arbeitsfrequenzen des portablen Frittierölsensors eingerichtet ist/sind. Von Vorteil ist dabei, dass Multifrequenzmessungen der frequenzabhängigen Permittivität durch Anfahren unterschiedlicher Arbeitsfrequenzen durchführbar sind. Hierdurch ist die Genauigkeit der Charakterisierung des Frittieröls verbesserbar.
Hierbei kann vorgesehen sein, dass mehrere, unterschiedlichen Arbeitsfrequenzen des portablen Frittierölsensors zugeordnete Kennlinien zur Verwendung in der portablen Auswerteeinheit hinterlegt sind. Somit sind frequenzabhängige Auswertungen von Ausgangssignalen des portablen Frittierölsensors ausführbar. Bevorzugt sind die anfahrbaren Arbeitsfrequenzen auf die anfahrbaren Arbeitsfrequenzen des stationären Frittierölmessgeräts abgestimmt. Die Datenverbindung kann in diesem Fall zur Übermittlung eines frequenzabhängigen Referenzwerts eingerichtet sein. Weiterhin weist ein Frittiereinrichtungs-Set eine Frittiereinrichtung gemäß der Erfindung, insbesondere wie zuvor beschrieben, und ein portables Frittierölmessgerät , insbesondere wie zuvor beschrieben, auf, wobei die Empfangs-Datenschnittstelle der Frittiereinrichtung zur Übertragung des Referenzwertes passend zu der Sende-Datenschnittstelle des portablen Frittierölmessgeräts ausgebildet ist. Von Vorteil ist dabei, dass das Frittiereinrichtungs-Set zwei voneinander unabhängige Möglichkeiten zur Bestimmung des Verbrauchtheitsgrades des Frittieröls aufweist, welche zur Kontrolle miteinander vergleichbar sind und verglichen werden.
Bei einer Ausgestaltung der Erfindung kann vorgesehen sein, dass die wenigstens eine, in der Fittiereinrichtung hinterlegte Kennlinie durch eine Offsetverschiebung in die in dem portablen Frittierölmessgerät hinterlegte, wenigstens eine Kennlinie überführbar ist. Von Vorteil ist dabei, dass eine Modifizierung der wenigstens einen Kennlinie in der Frittiereinrichtung besonders einfach durch Offsetverschiebung durchführbar ist.

Bei einer Ausgestaltung kann vorgesehen sein, dass der stationäre Frittierölsensor und der portable Frittierölsensor nach demselben physikalischen Messprinzip arbeiten. Beispielsweise kann vorgesehen sein, dass der stationäre Frittierölsensor und der portable Frittierölsensor zur Messung derselben physikalischen Größe oder miteinander vergleichbarer physikalischer Größen eingerichtet sind. Von Vorteil ist dabei, dass die ordnungsgemäße Funktion des stationären Frittierölsensors besonders einfach durch Vergleich von Messwerten mit dem portablen Frittierölsensor kontrollierbar ist.
Zur Lösung der genannten Aufgabe ist erfindungsgemäß bei einem Verfahren der eingangs beschriebenen Art vorgesehen, dass ein portabler Frittierölsensor eines portablen Frittierölmessgeräts in das Frittieröl eingetaucht wird, dass ein Messsignal des portablen Frittierölsensors in einen Referenzwert für eine Bewertungsgröße zur Charakterisierung des Frittieröls umgewandelt wird, dass der Referenzwert über wenigsten eine Datenschnittstelle an die stationäre Auswerteeinheit der Frittiereinrichtung übertragen wird, dass der Referenzwert mit einem Wert der wenigstens einen in der stationären Auswerteeinheit hinterlegten Kennlinie verglichen wird und dass eine Abweichung des Wertes von dem Referenzwert automatisiert ermittelt wird. Von Vorteil ist dabei, dass die ordnungsgemäße Funktion der Frittiereinrichtung, insbesondere des stationären Frittierölsensors, auf einfache Weise durch eine unabhängige Messung mit dem portablen Frittierölmessgerät automatisiert kontrollierbar ist.

Bei einer Ausgestaltung der Erfindung kann vorgesehen sein, dass der Wert der in der stationären Auswerteeinheit hinterlegten wenigstens einen Kennlinie aus einem Messsignal des stationären Frittierölsensors abgeleitet wird. Von Vorteil ist dabei, dass die ordnungsgemäße Funktion der Frittiereinrichtung im Betrieb kontrollierbar ist, indem der mit dem portablen Frittierölsensor gewonnene Referenzwert mit dem momentanen Wert zu dem momentanen Messsignal des stationären Frittierölsensors verglichen wird.

Bevorzugt ist vorgesehen, dass der portable Frittierölsensor und der stationäre Frittierölsensor während des Verfahrens dasselbe Frittieröl ausmessen. Besonders günstig ist es, wenn die jeweiligen Messvorgänge gleichzeitig durchgeführt werden.

Es kann vorgesehen sein, dass ein Hinweissignal generiert wird, wenn die Abweichung größer als ein Toleranzwert ist. Von Vorteil ist dabei, dass eine inakzeptable, nicht-ordnungsgemäße Funktion des stationären Frittierölsensors erkennbar und anzeigbar bzw. ausgebbar ist.

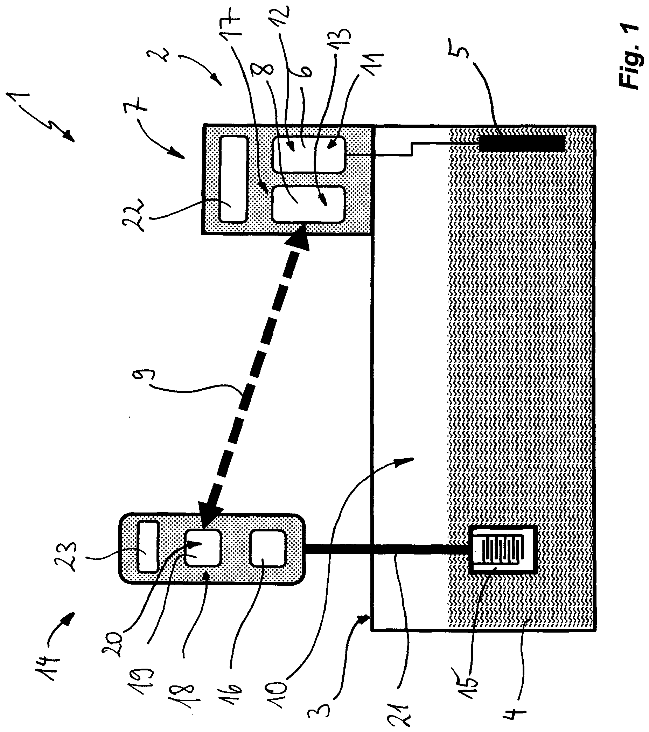

Bei einer Ausgestaltung der Erfindung kann vorgesehen sein, dass die in der stationären Auswerteeinheit hinterlegte wenigstens eine Kennlinie modifiziert wird, bis die Abweichung eines mit der modifizierten wenigstens einen Kennlinie aus dem Messsignal des stationären Fittierölsensors abgeleiteten oder gebildeten Wertes der Bewertungsgröße von dem Referenzwert kleiner als ein vorgegebener Schwellwert ist. Bevorzugt wird diese Modifizierung automatisiert durchgeführt. Von Vorteil ist dabei, dass eine Justage des stationären Frittierölsensors und der stationären Auswerteeinheit einfach und sogar automatisiert durchführbar ist, um eine definierte Funktionsweise der Frittiereinrichtung zu kontrollieren oder sogar wiederherzustellen.
Bevorzugt ist vorgesehen, dass der Schwellwert kleiner als der Toleranzwert ist. Der Schwellwert kann auch null betragen, so dass die Modifizierung der wenigstens einen Kennlinie durchgeführt wird, bis der Referenzwert gleich dem abgeleiteten Wert für die modifizierte wenigstens eine Kennlinie ist.
Es kann vorgesehen sein, dass die in der stationären Auswerteeinheit hinterlegte wenigstens einen Kennlinie durch eine Offset-Verschiebung modifiziert wird. Von Vorteil ist dabei, dass eine besonders einfache Form der Modifizierung, in welcher nur ein Parameter zur Offset-Verschiebung variiert wird, durchführbar ist.
Besonders günstig ist es, wenn in dem erfindungsgemäßen Verfahren eine erfindungsgemäße Frittiereinrichtung und/oder ein portables Frittierölmessgerät verwendet wird/werden. Werden sowohl eine erfindungsgemäße Frittiereinrichtung als auch ein portables Frittierölmessgerät eingesetzt, so ergeben sich sogar die Vorteile einer Verwendung eines Frittiereinrichtungs-Sets bei dem erfindungsgemäßen Verfahren.
Die Erfindung wird nun anhand eines Ausführungsbeispiels näher beschrieben, ist jedoch nicht auf das Ausführungsbeispiel beschränkt. Weitere Ausführungsbeispiele ergeben sich durch Kombination einzelner oder mehrerer Merkmale der Schutzansprüche untereinander und/oder mit einzelnen oder mehreren Merkmalen des Ausführungsbeispiels.
Es zeigt die einzige als
- Fig. 1: eine erfindungsgemäße Frittiereinrichtung in stark schematisierter Darstellung zur Erläuterung des erfindungsgemäßen Verfahrens.
Ein im Ganzen mit 1 bezeichnetes Frittiereinrichtungs-Set hat eine Frittiereinrichtung 2 zum Frittieren von Speisen.
Die Frittiereinrichtung 2 weist hierzu eine Frittierölbecken 3 auf, in welchem im Gebrauch das Frittieröl 4 aufgenommen ist.
Die Speisen werden somit durch Eintauchen in das Frittieröl 4 in dem Frittierölbecken 3 frittiert, wobei das Frittieröl 4 zuvor mit einer nicht weiter dargestellten, an sich bekannten Heizeinrichtung erhitzt wurde.
Zur Überwachung des Zustandes des Frittieröls 4 ist ein stationärer Frittierölsensor 5 vorgesehen, mit welche die Permittivität des Frittieröls 4 als elektrische Eigenschaft messbar ist. Hierbei kann der stationäre Frittierölsensor 5 den Realteil oder den Imaginärteil oder den Betrag oder eine sonstige Kombination von Realteil und Imaginärteil der Permittivität messen.

Es ist bekannt, dass sich das Frittieröl 4 nach einer gewissen Gebrauchszeit derart verändert, dass eine Veränderung der elektrischen Eigenschaften messbar ist. Somit kann mit dem stationären Frittierölsensor 5 erfasst werden, ob das Frittieröl 4 ausgetauscht werden muss.

Hierzu ist der stationäre Frittierölsensor 5 an eine stationäre Auswerteeinheit 6 angeschlossen, mit welcher die Messsignale des stationären Frittierölsensors 5 erfassbar und auswertbar sind.

Zur Auswertung ist in der stationären Auswerteeinheit 6 wenigstens eine Kennlinie hinterlegt, mit welcher das Messsignal des stationären Frittierölsensors 5 in eine Bewertungsgröße zur Charakterisierung des Frittieröls 4 umgewandelt werden kann. Somit wird in der stationären Auswerteeinheit 6 aus dem Messsignal ein Wert für die Bewertungsgröße abgeleitet oder gebildet. Im Ausführungsbeispiel ist dies eine Angabe des Anteils polarer Bestandteile im Frittieröl 4. Es ist bekannt, dass dieser Anteil elektrisch polarer Bestandteile mit zunehmender Gebrauchsdauer des Frittieröls 4 ansteigt, so dass ab einem bestimmten prozentualen Anteil derartiger polarer Bestandteile (TPM) ein Austausch des Frittieröls 4 gegen neues Frittieröl erforderlich ist.

Durch das Messsignal des stationären Frittierölsensors 5 wird in der Kennlinie ein Referenzpunkt definiert, an welchem die aktuelle Bewertungsgröße zur Charakterisierung des Frittieröls 4 entnommen wird.

Die stationäre Auswerteeinheit 6 ist als Teil eines stationären Frittierölmessgeräts 7 ausgebildet. Das stationäre Frittierölmessgerät 7 ist an dem Frittierölbecken 3 befestigt oder mit diesem verbunden.

An dem stationären Frittierölmessgerät 7 der Frittiereinrichtung 2 ist weiter eine Empfangseinheit 17 mit einer Empfangs-Datenschnittstelle 8 ausgebildet. Mit der Empfangs-Datenschnittstelle 8 ist über eine Datenverbindung 9 ein Referenzwert empfangbar. Diese wird im Messbetrieb der stationären Auswerteeinheit 6 empfangen.

Wie bereits erwähnt ist das stationäre Frittierölmessgerät 7 fest oder lösbar mit dem Frittierölbecken 3 verbunden.

Der stationäre Frittierölsensor 5 ist derart in dem Aufnahmeraum 10 des Frittierölbeckens 3 für das Frittieröl 4 angeordnet, dass der stationäre Frittierölsensor 5 - wie in Fig. 1 gezeigt - bei Normalbefüllung des Frittierölbeckens 3 in das Frittieröl 4 eingetaucht ist. Der stationäre Frittierölsensor 5 ist hierbei im Frittierölbecken 3 lösbar oder fest befestigt. Beispielsweise kann der Frittierölsensor 5 an der Zuleitung einer Heizung angebracht sein.

Die Empfangs-Datenschnittstelle 8 kann zur drahtgebundenen und/oder drahtlosen Datenübertragung eingerichtet sein. In Fig. 1 ist der Fall gezeigt, dass eine drahtlose Datenübertragung über die Datenverbindung 9 mit bluetooth oder einem anderen Protokoll durchführbar ist.

In der stationären Auswerteeinheit 6 ist eine Vergleichseinheit 11 ausgebildet, mit welcher der empfangene Referenzwert mit dem zuvor oder zeitlich parallel aus dem Messsignal des stationären Frittierölsensors 5 gebildeten Wertes für die Bewertungsgröße zur Charakterisierung des Frittieröls 4 vergleichbar ist und verglichen wird.

Die stationäre Auswerteeinheit 6 ist unter der Annahme eingerichtet, das im ordnungsgemäßen Betrieb des stationären Frittierölmessgeräts 7 der empfangene Referenzwert mit dem gebildeten Wert übereinstimmen soll. Jedoch kann sich die Funktion des stationären Frittierölsensors 5 aufgrund von Verschmutzungen, Alterungsprozessen und dergleichen, beispielsweise auch Beschädigungen, im Laufe der Zeit derart ändern, dass sich eine Abweichung des in der Umwandlung gebildeten Wertes von dem Referenzwert ergibt.

Ist die Abweichung dieser Werte voneinander größer als ein vorgegebener Toleranzwert, so bedeutet dies eine nicht-ordnungsgemäße Funktion des stationären Frittierölmessgeräts 7 und insbesondere des zugehörigen stationären Frittierölsensors 5.

Dies kann durch ein Hinweissignal, welches in der Vergleichseinheit 11 generiert wird, angezeigt und/oder ausgegeben und/oder protokolliert werden.

Im Ausführungsbeispiel gemäß Fig. 1 ist die erwähnte Kennlinie modifizierbar hinterlegt, so dass eine Veränderung der Kennlinie bei zu starker Abweichung des gebildeten oder berechneten Wertes von dem Referenzwert möglich ist und ausgeführt wird.

Hierzu weist die stationäre Auswerteeinheit 6 ein Mittel 12 zur Modifizierung der hinterlegten Kennlinie auf. Dies kann beispielsweise durch eine Änderung oder Anpassung von Parametern, welche die Kennlinie beschreiben, oder eine Offset-Verschiebung von beispielsweise tabellarisch hinterlegten Kennlinien-Werten erreicht sein.

Mit dem Mittel 12 zur Modifizierung der hinterlegten Kennlinie wird die modifizierte Kennlinie derart abgeändert, dass der Kennlinien-Wert der modifizierten Kennlinie am Referenzpunkt gleich dem empfangenen Referenzwert ist.

Da der Referenzpunkt durch das aktuelle Messsignal des stationären Frittierölsensors 5 definiert ist, ist jeder Punkt der Kennlinie als Referenzpunkt verwendbar und wird als Referenzpunkt definiert, wenn das Messsignal des stationären Frittierölsensors 5 diesen Punkt auszeichnet.

Der stationäre Frittierölsensor 5 ist in an sich bekannter Weise als kapazitiver Messsensor ausgebildet, mit welchem eine von der Permittivität des Frittieröls 4 abhängige physikalische Größe, beispielsweise eine Komponente dieser Permittivität, messbar ist und gemessen wird.

Das stationäre Frittierölmessgerät 7 umfasst ferner ein Mittel 13 zum Verbindungsaufbau der Datenverbindung 9.

Mit dem Mittel 13 ist beispielsweise manuell die Generierung oder Übertragung eines Referenzwertes auslösbar, welcher anschließend - wie beschrieben - mit der Empfangs-Datenschnittstelle 8 empfangbar ist.

Dieser Referenzwert ist mit einem im Ganzen mit 14 bezeichneten portablen Frittierölmessgerät erzeugbar.

Das portable Frittierölmessgerät weist hierzu einen portablen Frittierölsensor 15 auf, der zum Eintauchen in das mit Frittieröl 4 befüllte Frittierölbecken 3 der stationären Frittiereinrichtung 2 ausgebildet ist.

An diesen portablen Frittierölsensor 15 ist eine portable Auswerteeinheit 16 des portablen Frittierölmessgeräts 14 angeschlossen, mit welcher ein Messsignal des portablen Frittierölsensors 15 ausgelesen wird.

Dieses Messsignal wird in eine Bewertungsgröße zur Charakterisierung des Frittieröls 4 umgewandelt, indem eine in der portablen Auswerteeinheit 16 hinterlegte Kennlinie ausgewertet wird.

Auch der portable Frittierölsensor 15 ist in an sich bekannter Weise als kapazitiver Messsensor ausgebildet, mit welchem eine elektrische Eigenschaft des Frittieröls 4 messbar ist.

Der stationäre Frittierölsensor 5 und der portable Frittierölsensor 15 arbeiten nach demselben physikalischen Messprinzip und sind daher zur Messung derselben physikalischen Größe oder zumindest miteinander vergleichbarer physikalischer Größen eingerichtet.

Das portable Frittierölmessgerät 14 weist eine Sendeeinheit 18 auf, welche eine Sende-Datenschnittstelle 19 bereitstellt.

Die Sende-Datenschnittstelle 19 ist so auf die Empfangs-Datenschnittstelle 8 der Empfangseinheit 17 des stationären Frittierölmessgeräts 7 abgestimmt, dass beide Schnittstellen die Datenverbindung 9 definieren und bereitstellen.

Nach Betätigung des Mittels 13 zum Verbindungsaufbau an der Empfangseinheit 17 oder eines Mittels 20 zum Verbindungsaufbau an der Sendeeinheit 18 wird die Datenverbindung 9 aufgebaut, und die Sendeeinheit 18 sendet über die Sende-Datenschnittstelle 19 und die Empfangs-Datenschnittstelle 8 an die Empfangseinheit 17 den zu dem Messsignal des portablen Frittierölsensors 15 gebildeten Wert der Bewertungsgröße als Referenzwert.

Auf diese Weise ist in der Vergleichseinheit 11 des stationären Frittierölmessgeräts 7 die Messung mit dem portablen Frittierölsensor 15 mit der Messung mit dem stationären Frittierölsensor 5 vergleichbar, indem der über die Datenverbindung 9 übermittelte Referenzwert mit dem in der Auswerteeinheit 6 aktuell gebildeten Wert für die Bewertungsgröße zur Charakterisierung des Frittieröls 4 verglichen wird.

Eine Abweichung dieser Werte voneinander zeigt ein unterschiedliches Funktionieren des stationären Frittierölmessgeräts 7 einerseits und des portablen Frittierölmessgeräts 14 andererseits an.

Dieses unterschiedliche Funktionieren kann als Hinweis darauf gedeutet werden, dass die ordnungsgemäße Funktion des stationären Frittierölmessgeräts 7 im Laufe der Zeit beeinträchtigt wurde. Dies kann durch das bereits erwähnte Hinweissignal angezeigt oder ausgegeben werden. Durch eine entsprechende Nachführung der in der Auswerteeinheit 6 hinterlegten Kennlinie kann die Funktion des stationären Frittierölmessgeräts 7 auf die Funktion des portablen Frittierölmessgräts 14 abgeglichen werden.

Diese Abänderung der Kennlinie geschieht im Ausführungsbeispiel durch Offset-Verschiebung solange, bis der zu dem Messsignal des stationären Frittierölsensors 5 errechnete Wert der Bewertungsgröße nur noch um einen vorgegebenen Schwellwert, der auch null sein kann, von dem übermittelten Referenzwert abweicht.

Es sei noch erwähnt, dass das portable Frittierölmessgerät 14 ein stabförmiges Verbindungelement 21 aufweist, das zur Befestigung des portablen Frittierölsensors 15 an der portablen Auswerteeinheit 16 dient. Auf diese Weise kann der portable Frittierölsensor 15 in das heiße Frittieröl 4 eingetaucht werden, ohne dass die Elektronik der Auswerteeinheit 16 Schaden nimmt.

Das stationäre Frittierölmessgerät 7 weist ein Display 22 auf, mit welchem der aus dem Messsignal des stationären Frittierölsensors 5 mit Hilfe der hinterlegten Kennlinie gebildete Wert und/oder das zuvor erwähnte Hinweissignal anzeigbar ist. Zusätzlich können an dem Display 22 Informationen zu der Datenverbindung 9 angezeigt werden.

Das portable Frittierölmessgerät 14 weist ebenfalls ein Display 23 auf, mit welchem der aus dem Messsignal des portablen Frittierölsensors 15 mit Hilfe der im portablen Frittierölmessgerät 14 hinterlegten Kennlinie berechnete Wert anzeigbar ist und angezeigt wird. Somit ist das portable Frittierölmessgerät 14 auch in bekannter Weise als automatisches Frittierölmessgrät verwendbar. Durch die zusätzliche, neuartige Ausbildung und Verwendung einer Sendeeinheit 18 an dem portablen Frittierölmessgerät 14 ist der Funktionsumfang dieses portablen Frittierölmessgeräts 14 auf die Kontrolle der ordnungsgemäßen Funktion einer Frittiereinrichtung 2 ausdehnbar.

Zusätzlich können der stationäre Frittierölsensor 5 und die stationäre Auswerteeinheit 6 zum Betrieb bei unterschiedlichen Arbeitsfrequenzen des stationären Frittierölsensors 5 eingerichtet sein. Somit sind Multifrequenzmessungen der frequenzabhängigen Permittivität ausführbar. Beispielsweise können so unterschiedliche Arbeitsfrequenzen des Frittierölsensor 5 angefahren werden, bei denen dann jeweils ein Wert der Bewertungsgröße ermittelt wird.

Hierzu sind mehrere Kennlinien zur Verwendung in der stationären Auswerteeinheit 6 hinterlegt, die jeweils unterschiedlichen Arbeitsfrequenzen des Frittierölsensors 5 zugeordnet sind. Es wird dann automatisch jeweils die Kennlinie zu der momentanen Arbeitsfrequenz ausgewählt und ausgewertet.

In analoger Weise sind der portable Frittierölsensor 15 und die portable Auswerteeinheit 16 zur Durchführung von Multifrequenzmessungen eingerichtet. Dies erlaubt einen frequenzabhängigen Abgleich der Kennlinien im stationären Frittierölmessgerät 7 gegen einen jeweils zugehörigen, frequenzbezogenen Referenzwert von dem portablen Frittierölmessgerät 7.

Bei der Frittiereinrichtung 2 mit einem in Frittieröl 4 eines Frittierölbeckens 3 eintauchenden, stationären Frittierölsensor 5 wird vorgeschlagen, zur Kontrolle des stationären Frittierölsensors 5 eine Empfangseinheit 17 mit einer Empfangs-Datenschnittstelle 8 auszubilden, über welche ein mit einem portablen Frittierölmessgerät 14, dessen portabler Frittierölsensor 15 in das Frittieröl 4 im Frittierölbecken 3 zur Messung eingetaucht ist, zuvor gewonnener Referenzwert übertragbar ist, um eine in der stationären Auswerteeinheit 6 der Frittiereinrichtung 2 hinterlegte Kennlinie derart anzupassen, dass die stationäre Auswerteeinheit 6 einen Wert für eine Bewertungsgröße zur Charakterisierung des Frittieröls 4 errechnet, welcher in Übereinstimmung mit dem Referenzwert von dem portablen Frittierölmessgerät 14 ist (Fig. 1).

## Patentansprüche

1. Frittiereinrichtung (2), mit einem Frittierölbecken (3), das zur Aufnahme von Frittieröl (4) eingerichtet ist, einem stationären Frittierölsensor (5), welcher zur Messung einer elektrischen Eigenschaft des in dem Frittierölbecken (3) aufgenommenen Frittieröls (4) eingerichtet ist, und einer an den stationären Frittierölsensor (5) angeschlossenen stationären Auswerteeinheit (6), mit welcher ein Messsignal des stationären Frittierölsensors (5) automatisch mittels wenigstens einer in der stationären Auswerteeinheit (6) hinterlegten Kennlinie in eine Bewertungsgröße zur Charakterisierung des Frittieröls (4) umwandelbar ist, **wobei** in der wenigstens einen Kennlinie wenigstens ein Referenzpunkt definiert oder definierbar ist und dass eine Empfangseinheit (17) ausgebildet ist, welche eine Empfangs-Datenschnittstelle (8) zum Empfang eines Referenzwertes für den Referenzpunkt bereitstellt, wobei die Empfangs-Datenschnittstelle (8) zur drahtlosen Datenübertragung eingerichtet ist und die stationäre Auswerteeinheit (6) ein Mittel (12) zur Modifizierung der wenigstens einen hinterlegten Kennlinie aufweist, mit welchem die wenigstens eine Kennlinie derart modifizierbar ist, dass ein Kennlinienwert der modifizierten wenigstens einen Kennlinie am Referenzpunkt gleich dem Referenzwert ist.

2. Frittiereinrichtung (2) nach Anspruch 1, **dadurch gekennzeichnet, dass** der stationäre Frittierölsensor (5) in einem Aufnahmeraum (10) des Frittierölbeckens (3) für das Frittieröl (4) angeordnet ist.

3. Frittiereinrichtung (2) nach Anspruch 1 oder 2, **dadurch gekennzeichnet, dass** der stationäre Frittierölsensor (5) im Frittierölbecken (3) befestigt ist.

4. Frittiereinrichtung (2) nach einem der Ansprüche 1 bis 3, **dadurch gekennzeichnet, dass** der Referenzwert während eines Messbetriebs der stationären Auswerteeinheit (6) empfangbar ist.

5. Frittiereinrichtung (2) nach einem der Ansprüche 1 bis 4, **dadurch gekennzeichnet, dass** jeder Punkt der wenigstens einen Kennlinie als Referenzpunkt verwendbar oder definierbar ist.

6. Frittiereinrichtung (2) nach einem der Ansprüche 1 bis 5, **dadurch gekennzeichnet, dass** der Referenzpunkt durch das aktuelle Messsignal des stationären Frittierölsensors (5) definiert ist.

7. Frittiereinrichtung (2) nach einem der Ansprüche 1 bis 6, **dadurch gekennzeichnet, dass** eine Vergleichseinheit (11) ausgebildet ist, die zur Generierung eines Hinweissignals, wenn der Referenzwert um wenigstens einen Toleranzwert von einem Wert der hinterlegten wenigstens einen Kennlinie am Referenzpunkt und/oder von einem aus einem Messsignal des stationären Frittierölsensors (5) gebildeten Wert der Bewertungsgröße abweicht, eingerichtet ist.

8. Frittiereinrichtung (2) nach einem der Ansprüche 1 bis 7, **dadurch gekennzeichnet, dass** die wenigstens eine Kennlinie modifizierbar hinterlegt ist.

9. Frittiereinrichtung (2) nach einem der Ansprüche 1 bis 8, **dadurch gekennzeichnet, dass** der stationäre Frittierölsensor (5) und/oder die stationäre Auswerteeinheit (6) zum Betrieb bei unterschiedlichen Arbeitsfrequenzen des stationären Frittierölsensors (5) eingerichtet ist/sind und/oder dass mehrere, unterschiedlichen Arbeitsfrequenzen des Frittierölsensors (5) zugeordnete Kennlinien zur Verwendung in der stationären Auswerteeinheit (6) hinterlegt sind.

10. Frittiereinrichtung (2) nach einem der Ansprüche 1 bis 9, **dadurch gekennzeichnet, dass** mit dem stationären Frittierölsensor (5) eine von der Permittivität des Frittieröls (4) abhängige physikalische Größe messbar ist.

11. Frittiereinrichtung (2) nach einem der Ansprüche 1 bis 10, **dadurch gekennzeichnet, dass** der stationäre Frittierölsensor (5) als kapazitiver Messsensor ausgebildet ist.

12. Frittiereinrichtung (2) nach einem der Ansprüche 1 bis 11, **dadurch gekennzeichnet, dass** die Frittiereinrichtung (2) ein Mittel(13) zum Verbindungsaufbau einer Datenverbindung (9) über die Empfangs-Datenschnittstelle (8) aufweist.

13. Verfahren zur Kontrolle einer Frittiereinrichtung (2), wobei die Frittiereinrichtung (2) einen stationären Frittierölsensor (5), welcher zur Messung einer elektrischen Eigenschaft eines in einem Frittierölbecken (3) der Frittiereinrichtung (2) aufgenommenen Frittieröls (4) eingerichtet ist, aufweist und wobei in einer an den stationären Frittierölsensor (5) angeschlossenen stationären Auswerteeinheit (6) ein Messsignal des stationären Frittierölsensors (5) automatisch mittels wenigstens einer in der stationären Auswerteeinheit (6) hinterlegten Kennlinie in eine Bewertungsgröße zur Charakterisierung des Frittieröls (4) umgewandelt wird, **dadurch gekennzeichnet, dass** ein vom stationären Frittierölsensor (5) unabhängiger portabler Frittierölsensor (15) eines portablen Frittierölmessgeräts (14) in das Frittieröl (4) eingetaucht wird, dass ein Messsignal des portablen Frittierölsensors (15) in einen Referenzwert für eine Bewertungsgröße zur Charakterisierung des Frittieröls (4) umgewandelt wird, dass der Referenzwert über wenigstens eine Datenschnittstelle (8, 19) an die stationäre Auswerteeinheit (6) der Frittiereinrichtung (2) übertragen wird, dass der Referenzwert mit einem Wert der in der stationären Auswerteeinheit (6) hinterlegten wenigstens einen Kennlinie verglichen wird und dass eine Abweichung des Wertes von dem Referenzwert automatisiert ermittelt wird.

14. Verfahren nach Anspruch 13, **dadurch gekennzeichnet, dass** der Wert der in der stationären Auswerteeinheit (6) hinterlegten wenigstens einen Kennlinie aus einem Messsignal des stationären Frittierölsensors (5) abgeleitet wird.

15. Verfahren nach Anspruch 13 oder 14, **dadurch gekennzeichnet, dass** ein Hinweissignal generiert wird, wenn die Abweichung größer als ein Toleranzwert ist.

16. Verfahren nach einem der Ansprüche 13 bis 15, **dadurch gekennzeichnet, dass** die in der stationären Auswerteeinheit (6) hinterlegte wenigstens eine Kennlinie vorzugsweise automatisiert modifiziert wird, bis die Abweichung eines mit der modifizierten wenigstens eine Kennlinie aus dem Messsignal des stationären Frittierölsensors (5) abgeleiteten Wertes der Bewertungsgröße von dem Referenzwert kleiner als ein vorgegebener Schwellwert ist.

17. Verfahren nach einem der Ansprüche 13 bis 16, **dadurch gekennzeichnet, dass** der Schwellwert kleiner als der Toleranzwert ist.

18. Verfahren nach einem der Ansprüche 13 bis 17, **dadurch gekennzeichnet, dass** die in der stationären Auswerteeinheit hinterlegte wenigstens eine Kennlinie durch eine Offset-Verschiebung modifiziert wird.

19. Verfahren nach einem der Ansprüche 13 bis 18, **dadurch gekennzeichnet, dass** eine Frittiereinrichtung (2) nach einem der Ansprüche 1 bis 12 verwendet wird.

## Claims

1. Deep-frying device (2) having a deep-frying oil reservoir (3) which is arranged to receive deep-frying oil (4), a stationary deep-frying oil sensor (5) which is arranged to measure an electrical property of the deep-frying oil (4) received in the deep-frying oil reservoir (3), and a stationary evaluation unit (6) which is connected to the stationary deep-frying oil sensor (5) and with which a measurement signal of the stationary deep-frying oil sensor (5) is automatically converted into an evaluation variable for characterising the deep-frying oil (4) by means of at least one characteristic curve stored in the stationary evaluation unit (6), wherein at least one reference point is, or can be, defined in the at least one characteristic curve, and that a receiving unit (17) is formed which provides a receiving data interface (8) for receiving a reference value for the reference point, wherein the receiving data interface (8) is arranged for wireless data transmission and the stationary evaluation unit (6) comprises a means (12) for modifying the at least one stored characteristic curve, with which means the at least one characteristic curve can be modified in such a way that a characteristic curve value of the modified at least one characteristic curve at the reference point is the same as the reference value.

2. Deep-frying device (2) as claimed in claim 1, **characterised in that** the stationary deep-frying oil sensor (5) is disposed in a receiving space (10) of the deep-frying oil reservoir (3) for the deep-frying oil (4).

3. Deep-frying device (2) as claimed in claim 1 or 2, **characterised in that** the stationary deep-frying oil sensor (5) is fastened in the deep-frying oil reservoir (3).

4. Deep-frying device (2) as claimed in any one of claims 1 to 3, **characterised in that** the reference value can be received during a measurement operation of the stationary evaluation unit (6).

5. Deep-frying device (2) as claimed in any one of claims 1 to 4, **characterised in that** each point of the at least one characteristic curve can be used or defined as a reference point.

6. Deep-frying device (2) as claimed in any one of claims 1 to 5, **characterised in that** the reference point is defined by the current measurement signal of the stationary deep-frying oil sensor (5).

7. Deep-frying device (2) as claimed in any one of claims 1 to 6, **characterised in that** a comparison unit (11) is formed which is arranged to generate an indicating signal if the reference value deviates by at least one tolerance value from a value of the stored at least one characteristic curve at the reference point and/or from a value of the evaluation variable, formed from a measurement signal of the stationary deep-frying oil sensor (5).

8. Deep-frying device (2) as claimed in any one of claims 1 to 7, **characterised in that** the at least one characteristic curve is stored in a modifiable manner.

9. Deep-frying device (2) as claimed in any one of claims 1 to 8, **characterised in that** the stationary deep-frying oil sensor (5) and/or the stationary evaluation unit (6) is/are arranged to operate at different operating frequencies of the stationary deep-frying oil sensor (5) and/or that a plurality of characteristic curves assigned to different operating frequencies of the deep-frying oil sensor (5) are stored for use in the stationary evaluation unit (6).

10. Deep-frying device (2) as claimed in any one of claims 1 to 9, **characterised in that** the stationary deep-frying oil sensor (5) can measure a physical variable dependent on the permittivity of the deep-frying oil (4).

11. Deep-frying device (2) as claimed in any one of claims 1 to 10, **characterised in that** the stationary deep-frying oil sensor (5) is designed as a capacitive measuring sensor.

12. Deep-frying device (2) as claimed in any one of claims 1 to 11, **characterised in that** the deep-frying device (2) comprises a means (13) for setting up a data connection (9) via the receiving data interface (8).

13. Method for monitoring a deep-frying device (2), wherein the deep-frying device (2) comprises a stationary deep-frying oil sensor (5) which is arranged to measure an electrical property of a deep-frying oil (4) received in a deep-frying oil reservoir (3) of the deep-frying device (2), and wherein, in a stationary evaluation unit (6) connected to the stationary deep-frying oil sensor (5), a measurement signal of the stationary deep-frying oil sensor (5) is automatically converted into an evaluation variable for characterising the deep-frying oil (4) by means of at least one characteristic curve stored in the stationary evaluation unit (6), **characterised in that** a portable deep-frying oil sensor (15) of a portable deep-frying oil measuring device (14) - this deep-frying oil sensor being independent of the stationary deep-frying oil sensor (5) - is immersed in the deep-frying oil (4), that a measurement signal of the portable deep-frying oil sensor (15) is converted into a reference value for an evaluation variable for characterising the deep-frying oil (4), that the reference value is transmitted to the stationary evaluation unit (6) of the deep-frying device (2) via at least one data interface (8, 19), that the reference value is compared with a value of the at least one characteristic curve stored in the stationary evaluation unit (6), and that a deviation of the value from the reference value is automatically determined.

14. Method as claimed in claim 13, **characterised in that** the value of the at least one characteristic curve stored in the stationary evaluation unit (6) is derived from a measurement signal of the stationary deep-frying oil sensor (5).

15. Method as claimed in claim 13 or 14, **characterised in that** an indicating signal is generated if the deviation is greater than a tolerance value.

16. Method as claimed in any one of claims 13 to 15, **characterised in that** the at least one characteristic curve stored in the stationary evaluation unit (6) is preferably automatically modified until the deviation of a value of the evaluation variable - which is derived from the measurement signal of the stationary deep-frying oil sensor (5) with the modified at least one characteristic curve - from the reference value is less than a preset threshold value.

17. Method as claimed in any one of claims 13 to 16, **characterised in that** the threshold value is lower than the tolerance value.

18. Method as claimed in any one of claims 13 to 17, **characterised in that** the at least one characteristic curve stored in the stationary evaluation unit is modified by an offset shift.

19. Method as claimed in any one of claims 13 to 18, **characterised in that** the deep-frying device (2) as claimed in any one of claims 1 to 12 is used.

## Revendications

1. Dispositif de friture (2), avec une cuve d'huile de friture (3), qui est conçue pour contenir de l'huile de friture (4), avec un détecteur d'huile de friture stationnaire (5), qui est conçu pour la mesure d'une propriété électrique de l'huile de friture (4) contenue dans la cuve d'huile de friture (3), et avec une unité d'évaluation stationnaire (6) reliée au détecteur d'huile de friture stationnaire (5), avec laquelle un signal de mesure du détecteur d'huile de friture stationnaire (5) peut être converti automatiquement au moyen d'au moins une courbe caractéristique stockée dans l'unité d'évaluation stationnaire (6) en une grandeur d'évaluation pour la caractérisation de l'huile de friture (4), dans lequel au moins un point de référence est ou peut être défini dans ladite au moins une courbe caractéristique et il se trouve une unité de réception (17), qui procure une interface de données de réception (8) pour la réception d'une valeur de référence pour le point de référence, dans lequel l'interface de données de réception (8) est conçue pour la transmission de données sans fil et l'unité d'évaluation stationnaire (6) présente un moyen (12) pour la modification de ladite au moins une courbe caractéristique stockée, avec lequel ladite au moins une courbe caractéristique stockée peut être modifiée de telle manière qu'une valeur de courbe caractéristique de ladite au moins une courbe caractéristique modifiée au point de référence soit égale à la valeur de référence.

2. Dispositif de friture (2) selon la revendication 1, **caractérisé en ce que** le détecteur d'huile de friture stationnaire (5) est disposé dans une chambre de réception (10) de la cuve d'huile de friture (3) pour l'huile de friture (4).

3. Dispositif de friture (2) selon la revendication 1 ou 2, **caractérisé en ce que** le détecteur d'huile de friture stationnaire (5) est fixé dans la cuve d'huile de friture (3).

4. Dispositif de friture (2) selon l'une quelconque des revendications 1 à 3, **caractérisé en ce que** la valeur de référence peut être reçue pendant une opération de mesure de l'unité d'évaluation stationnaire (6).

5. Dispositif de friture (2) selon l'une quelconque des revendications 1 à 4, **caractérisé en ce que** chaque point de ladite au moins une courbe caractéristique peut être utilisé ou défini comme point de référence.

6. Dispositif de friture (2) selon l'une quelconque des revendications 1 à 5, **caractérisé en ce que** le point de référence est défini par le signal de mesure actuel du détecteur d'huile de friture stationnaire (5).

7. Dispositif de friture (2) selon l'une quelconque des revendications 1 à 6, **caractérisé en ce qu'**il se trouve une unité de comparaison (11), qui est conçue pour générer un signal d'information, lorsque la valeur de référence s'écarte d'au moins une valeur de tolérance d'une valeur de ladite au moins une courbe caractéristique stockée au point de référence et/ou d'au moins une valeur de la grandeur d'évaluation formée à partir d'un signal de mesure du détecteur d'huile de friture stationnaire (5).

8. Dispositif de friture (2) selon l'une quelconque des revendications 1 à 7, **caractérisé en ce que** ladite au moins une courbe caractéristique est stockée de façon modifiable.

9. Dispositif de friture (2) selon l'une quelconque des revendications 1 à 8, **caractérisé en ce que** le détecteur d'huile de friture stationnaire (5) et/ou l'unité d'évaluation stationnaire (6) est/sont conçu(e)(s) pour le fonctionnement à différentes fréquences de travail du détecteur d'huile de friture stationnaire (5) et/ou **en ce que** plusieurs courbes caractéristiques associées à différentes fréquences de travail du détecteur d'huile de friture (5) sont stockées en vue de leur utilisation dans l'unité d'évaluation stationnaire (6).

10. Dispositif de friture (2) selon l'une quelconque des revendications 1 à 9, **caractérisé en ce qu'**une grandeur physique dépendant de la permittivité de l'huile de friture (4) peut être mesurée avec le détecteur d'huile de friture stationnaire (5).

11. Dispositif de friture (2) selon l'une quelconque des revendications 1 à 10, **caractérisé en ce que** le détecteur d'huile de friture stationnaire (5) est un détecteur de mesure capacitif.

12. Dispositif de friture (2) selon l'une quelconque des revendications 1 à 11, **caractérisé en ce que** le dispositif de friture (2) présente un moyen (13) pour l'établissement de liaison d'une liaison de données (9) via l'interface de données de réception (8).

13. Procédé de commande d'un dispositif de friture (2), dans lequel le dispositif de friture (2) présente un détecteur d'huile de friture stationnaire (5), qui est conçu pour la mesure d'une propriété électrique de l'huile de friture (4) contenue dans la cuve d'huile de friture (3) du dispositif de friture (2) et dans lequel on convertit automatiquement dans une unité d'évaluation stationnaire (6) reliée au détecteur d'huile de friture stationnaire (5) un signal de mesure du détecteur d'huile de friture stationnaire (5), au moyen d'au moins une courbe caractéristique stockée dans l'unité d'évaluation stationnaire (6), en une grandeur d'évaluation pour la caractérisation de l'huile de friture (4), **caractérisé en ce que** l'on plonge un détecteur d'huile de friture portable (15) d'un appareil de mesure d'huile de friture portable (14) indépendant du détecteur d'huile de friture stationnaire (5) dans l'huile de friture (4), **en ce que** l'on convertit un signal de mesure du détecteur d'huile de friture portable (15) en une valeur de référence pour une grandeur d'évaluation en vue de la caractérisation de l'huile de friture (4), **en ce que** l'on transmet la valeur de référence via au moins une interface de données (8, 19) à l'unité d'évaluation stationnaire (6) du dispositif de friture (2), **en ce que** l'on compare la valeur de référence avec une valeur de ladite au moins une courbe caractéristique stockée dans l'unité d'évaluation stationnaire (6) et **en ce que** l'on détermine automatiquement un écart de la valeur de la grandeur d'évaluation déduite par rapport à la valeur de référence.

14. Procédé selon la revendication 13, **caractérisé en ce que** l'on déduit la valeur de ladite au moins une courbe caractéristique stockée dans l'unité d'évaluation stationnaire (6) d'un signal de mesure du détecteur d'huile de friture stationnaire (5).

15. Procédé selon la revendication 13 ou 14, **caractérisé en ce que** l'on génère un signal d'information, lorsque l'écart est supérieur à une valeur de tolérance.

16. Procédé selon l'une quelconque des revendications 13 à 15, **caractérisé en ce que** l'on modifie de préférence automatiquement ladite au moins une courbe caractéristique stockée dans l'unité d'évaluation stationnaire (6), jusqu'à ce que l'écart d'une valeur de la grandeur d'évaluation déduite du signal de mesure du détecteur d'huile de friture stationnaire (5) avec ladite au moins une courbe caractéristique modifiée par rapport à la valeur de référence soit inférieur à une valeur de seuil prédéterminée.

17. Procédé selon l'une quelconque des revendications 13 à 16, **caractérisé en ce que** la valeur de seuil est inférieure à la valeur de tolérance.

18. Procédé selon l'une quelconque des revendications 13 à 17, **caractérisé en ce que** l'on modifie ladite au moins une courbe caractéristique stockée dans l'unité d'évaluation stationnaire au moyen d'un glissement offset.

19. Procédé selon l'une quelconque des revendications 13 à 18, **caractérisé en ce que** l'on utilise un dispositif de friture (2) selon l'une quelconque des revendications 1 à 12.
